# DEMANDE DE BREVET EUROPEEN

(11) **EP 0 955 007 A1**
(43) Date de publication de la demande: **10.11.1999**
(21) Numéro de dépôt: 99410040.2
(22) Date de dépôt: 28.04.1999
(51) Int. Cl.: A61B 5/00, A61B 5/0245

(54) **Dispositif de détection et de transmission à distance d'une alarme du rythme cardiaque d'une personne à surveiller**

(30) Priorité: 05.05.1998 FR 9805904
(71) Demandeur: S.U.M.A.T.E.L., 73540 La Bathie (FR)
(72) Inventeur: Gros, Daniel, 73540 La Bathie (FR)
(74) Mandataire: Hecké, Gérard

(57) **Abrégé**

Un dispositif de détection et de transmission de l'alarme du rythme cardiaque d'une personne est assurée au moyen :
- d'un premier module de détection (12) et d'émission destiné à délivrer un signal de mesure (10,11) du rythme cardiaque à un convertisseur tension-fréquence (18) connecté à un premier circuit émetteur (20) à courte portée,
- d'un deuxième module relais (14) placé à proximité du premier module, et comportant un premier circuit récepteur (24) à très courte portée, un dispositif de réglage (30) pour ajuster un seuil haut, et un seuil bas du rythme cardiaque, et un transmetteur (32) capable de délivrer un signal sonore d'alarme dès que le signal de fréquence cardiaque dépasse le seuil haut, ou descend en-dessous du seuil bas,
- d'un système de télétransmission comprenant un deuxième circuit émetteur à longue portée, piloté par un détecteur acoustique (34),
- et d'un troisième module (16) de réception du signal d'alarme.

## Description

L'invention est relative à un dispositif de détection et de transmission d'une alarme du rythme cardiaque d'une personne au moyen d'un premier module de détection et d'émission comprenant un appareil portatif à capteur de mesure fixé sur le corps de la personne, et destiné à délivrer un signal de mesure du rythme cardiaque à un convertisseur tension-fréquence.

Les appareils portatifs à usage domestique pour la surveillance du rythme cardiaque de certaines personnes, par exemple des sportifs, communiquent directement par liaison radioélectrique avec un appareil récepteur de surveillance. La portée limitée de ces appareils connus est de l'ordre de 90 cm.

L'objet de l'invention consiste à réaliser un dispositif de surveillance fiable du rythme cardiaque d'une personne avec retransmission d'un signal d'alarme sur une longue portée.

Le dispositif de détection et de transmission selon l'invention est caractérisé en ce que :
- le convertisseur tension-fréquence est connecté à un premier circuit émetteur radioélectrique à très courte portée, destiné à émettre un signal de fréquence cardiaque au moyen d'une première antenne émettrice,
- un deuxième module relais est placé à proximité du premier module, et comporte un premier circuit récepteur radioélectrique associé à une première antenne réceptrice à très courte portée, susceptible de capter le signal émis par la première antenne émettrice, un écran de visualisation pour afficher en lecture directe les pulsations du rythme cardiaque, un dispositif de réglage pour ajuster un seuil haut, et un seuil bas du rythme cardiaque, et un transmetteur capable de délivrer un signal sonore d'alarme dès que le signal de fréquence cardiaque dépasse le seuil haut, ou descend en-dessous du seuil bas,
- un système de télétransmission est logé dans le deuxième module relais et comprend un deuxième circuit émetteur radioélectrique à longue portée, piloté par un détecteur acoustique sensible au signal sonore d'alarme, et associé à une deuxième antenne émettrice,
- un troisième module de réception du signal d'alarme, est doté d'un deuxième circuit récepteur radioélectrique à longue portée, associé à une deuxième antenne réceptrice, et d'un circuit amplificateur branché à un indicateur sonore ou visuel, ledit troisième module portatif étant sous la surveillance d'une autre personne.

Selon un mode de réalisation préférentiel, le détecteur acoustique est formé par un microphone polarisé à électret coopérant avec un relais de commande d'un circuit inverseur de polarité associé au deuxième circuit émetteur. Le microphone polarisé à électret est inséré à l'entrée d'un circuit de traitement ayant des moyens de réglage du seuil de déclenchement du relais.

Selon une caractéristique de l'invention, le deuxième module relais comporte deux alimentations séparées, l'une étant affectée au premier circuit récepteur, et l'autre au système de télétransmission. La première alimentation comporte un premier régulateur de tension, et une pile de secours pour mémoriser les paramètres de réglage des seuils haut et bas, et pour afficher en permanence le rythme cardiaque indépendamment de l'état de la deuxième alimentation.

De préférence, le deuxième module relais est équipé d'un circuit test à bouton de manoeuvre destiné à simuler la commutation du relais pour vérifier l'émission du signal d'alarme à haute fréquence par le deuxième circuit émetteur du système de télétransmission.

D'autres avantages et caractéristiques ressortiront plus clairement de la description qui va suivre d'un mode de réalisation de l'invention, donné à titre d'exemple non limitatif et représenté aux dessins annexés dans lesquels :
- la figure 1 est une vue synoptique du premier module de détection et d'émission ;
- la figure 2 montre une vue synoptique du deuxième module relais ;
- la figure 3 représente une vue synoptique du troisième module de réception du signal d'alarme ;
- la figure 4 illustre les diagrammes de pulsations par minute en fonction de l'âge d'un nourrisson ;
- la figure 5 est une vue de détail du circuit électronique du deuxième module relais.

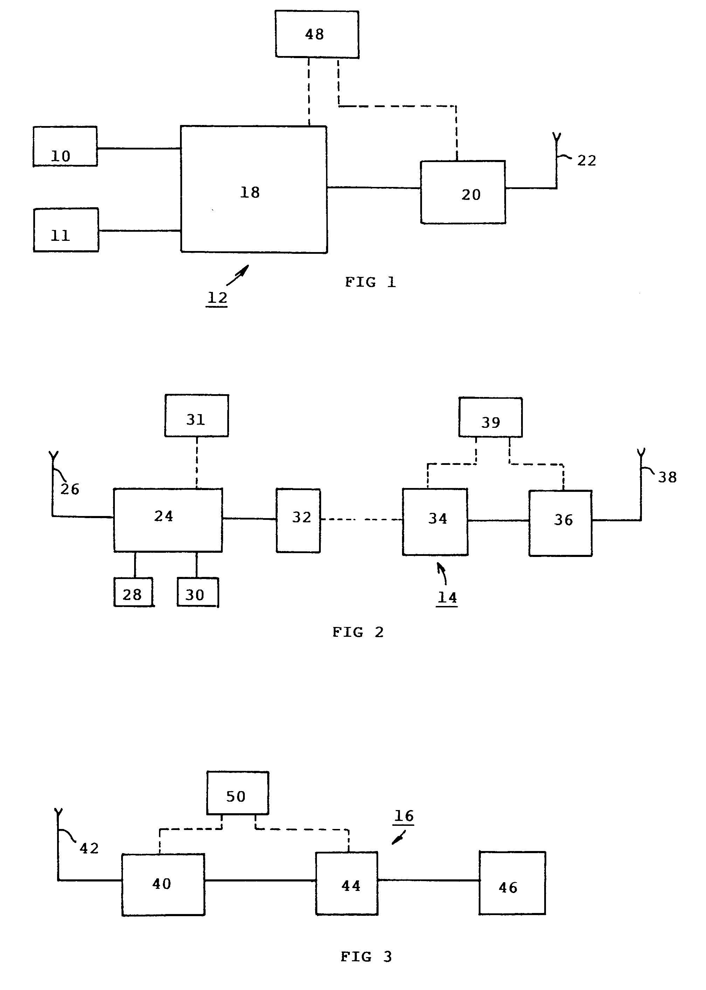

Sur les figures 1 à 3, le dispositif de détection et de transmission du rythme cardiaque d'une personne, est composé d'un premier module de détection et d'émission 12 (figure 1) porté par la personne dont le rythme cardiaque est à surveiller, d'un deuxième module relais 14 (figure 2) placé à proximité de la personne, et d'un troisième module de réception 16 de l'alarme (figure 3).

En référence à la figure 1, le premier module de détection et d'émission 12 est formé par une carte électronique comportant deux capteurs 10, 11 de mesure du rythme cardiaque, destinés à délivrer un signal de tension à un convertisseur tension-fréquence 18 associé à un premier circuit émetteur 20 radioélectrique à très courte portée. La carte est logée dans un étui thermorétractable équipé d'une couche de protection à base de mousse synthétique, de manière à former un coussin relié à deux électrodes destinées à venir en contact avec le corps de la personne à surveiller. Le premier circuit émetteur 20 est doté d'une première antenne émettrice 22.

Le deuxième module relais 14 de la figure 2, est disposé à une distance maximale de 90 cm de la personne équipée du premier module 12, et sert à retransmettre le signal émis par le premier circuit émetteur 20 à très courte portée vers le troisième module de réception 16 pouvant être éloigné d'une grande distance, par exemple de l'ordre de 700 m. La transmission intervient exclusivement en cas de dépassement d'un seuil haut, et d'un seuil bas du rythme cardiaque détecté.

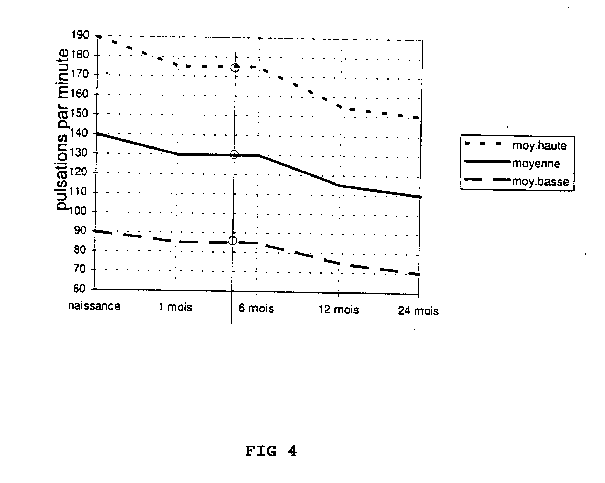

Le deuxième relais 14 comporte un premier circuit récepteur 24 radioélectrique associé à une première antenne réceptrice 26 à très courte portée, par exemple 1 m. Le premier circuit récepteur 24 capte le signal émis par l'antenne émettrice 22, et affiche en lecture directe le rythme cardiaque sur un écran de visualisation 28, par exemple un écran LCD à cristaux liquides. Un dispositif de réglage 30 permet d'ajuster le seuil haut et le seuil bas du rythme cardiaque. La figure 4 montre à titre d'exemple les diagrammes de pulsations par minute en fonction de l'âge d'un nourrisson. Ainsi à quatre mois, le seuil haut est de 175 pulsations par minute, et le seuil bas est de 85 pulsations par minute. Une première alimentation 31 à 3 volts est affectée au premier circuit récepteur 24, lequel affiche en permanence le rythme cardiaque avec rafraîchissement toutes les quatre secondes.

Le premier circuit récepteur 24 de fréquence cardiaque est pourvu d'un transmetteur 32 capable de délivrer un signal sonore d'alarme dès que le rythme cardiaque dépasse le seuil haut ou descend en-dessous du seuil bas. Le signal sonore est capté par un détecteur acoustique 34, lequel déclenche le système de télétransmission, formé par un deuxième circuit émetteur 36 à longue portée, lequel est associé à une deuxième antenne émettrice 38. La portée entre le deuxième circuit émetteur 36 et la personne porteuse du troisième module de réception 16 de l'alarme, est de 700 m environ. Une deuxième alimentation 39 indépendante de la première alimentation 31, est affectée au détecteur acoustique 34 et au deuxième circuit émetteur 36.

Le troisième module de réception 16 du signal d'alarme émis par la deuxième antenne émettrice 38 du module relais 14 comprend un deuxième circuit récepteur 40 radioélectrique à longue portée, associé à une deuxième antenne réceptrice 42. La sortie du deuxième circuit récepteur 40 est connectée à un amplificateur 44 à basse fréquence branchée à un indicateur 46, notamment un haut parleur qui se met à sonner dès réception d'un signal radio en provenance du module relais 14. Le troisième module de réception 16 est porté par la personne chargée de la surveillance, et comporte un bouton d'arrêt du signal sonore après réception du signal radio d'alarme, un bouton de commande général d'arrêt et de mise en service du module, et une diode de fonctionnement.

Le premier module et le troisième module ont chacun une alimentation de 3 volts à piles, ayant respectivement les références 48 et 50.

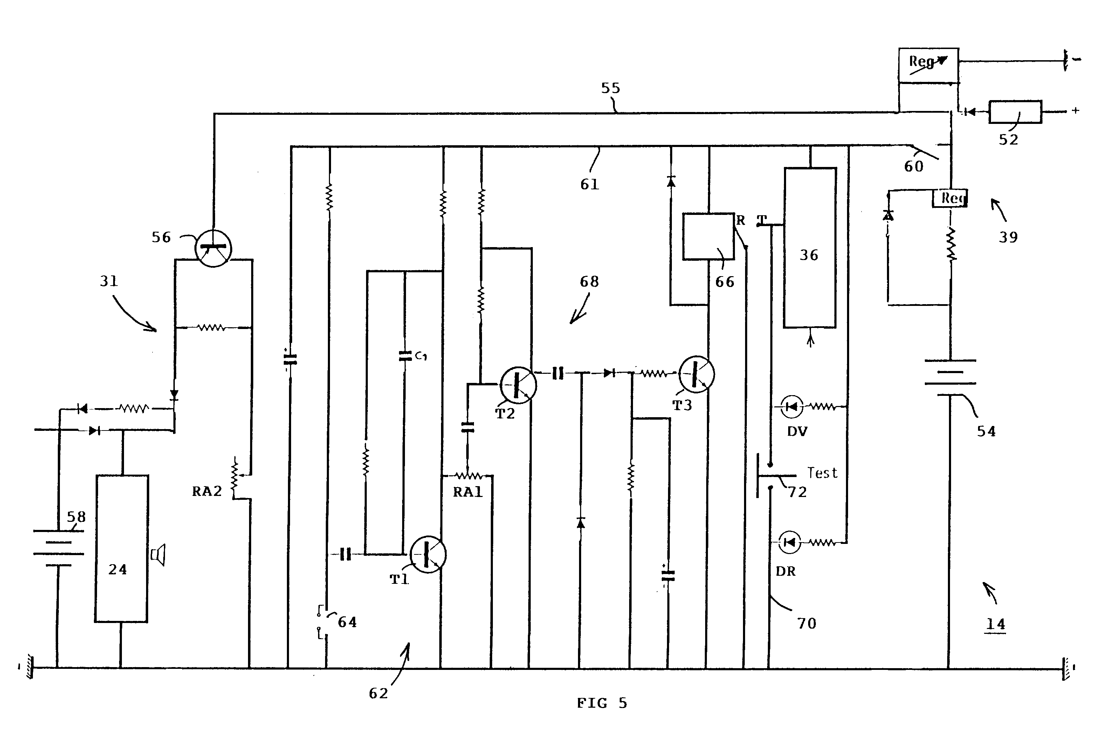

La figure 5 représente le schéma détaillé du deuxième module relais 14 du cardiofréquencemètre. Un chargeur de batterie séparé à 16 volts est susceptible d'alimenter le circuit du deuxième module à travers un coupe-circuit à fusible 52, et permet de charger un accumulateur 54 constituant la deuxième alimentation 39 pour un fonctionnement en autonome hors alimentation extérieure. La première alimentation 31 du premier circuit récepteur 24 comporte un premier régulateur de tension 56 relié au pôle positif de l'accumulateur 54, à travers une première ligne 55, et un accumulateur 58 de secours à 3 volts pour mémoriser les paramètres de réglage des seuils haut et bas. L'affichage du rythme cardiaque est ainsi visualisé en permanence quelque soit l'état ouvert ou fermé de l'interrupteur 60. Le réglage de la tension d'alimentation du premier régulateur s'opère au moyen du potentiomètre RA2.

La mise en service du module relais 14 est réalisée au moyen de l'interrupteur 60, et le signal de présence de tension est affiché sur une diode rouge DR. La fermeture de l'interrupteur 60 alimente à travers la ligne 61, à la fois le circuit de traitement 62, et le deuxième circuit émetteur 36 à haute fréquence (433Mhz) qui restent tous les deux en état de veille.

Le détecteur acoustique 34 est formé par un microphone polarisé à électret 64 qui capte le signal sonore issu du transmetteur 32 après dépassement de l'un des seuils haut ou bas. Le microphone 64 déclenche ensuite un relais de commande 66 par l'intermédiaire d'un circuit amplificateur 68 à transistors T1, T2, T3. Le relais 66 coopère avec un circuit inverseur 70 de polarité, associé au deuxième circuit émetteur 36. Le réglage du seuil de déclenchement du circuit de traitement acoustique 62 est assuré par le potentiomètre RA1, lequel est inséré entre le circuit collecteur de transistor T1 et le circuit de base du transistor T2. L'alarme est transmise au troisième module 16 dès que le relais 66 a commuté de la position R de repos vers la position T de travail.

Un circuit test 70 à bouton de manoeuvre 72 est inséré entre le pôle négatif de l'alimentation, et le plot T représentatif de la position de travail du relais de commande 66. La fermeture du bouton de manoeuvre 72 provoque l'allumage de la diode verte DV, et simule la commutation du relais 66 vers la position T entraînant l'émission du signal d'alarme à haute fréquence (433 MHz) vers la deuxième antenne réceptrice 42 du troisième module 16. Le test est effectué pour constater le bon fonctionnement de la télétransmission. Si la tension de l'accumulateur 54 est inférieure à 10 volts, le relais 66 reste en position R de repos, et inhibe la télétransmission de l'alarme .

Après mise en service, le deuxième module 14 reste toujours en veille, et sa consommation est de l'ordre de 8mA pour une autonomie de huit heures sans recharge. En fonctionnement d'alarme après commutation du relais 66 vers la position T de travail, la consommation est de l'ordre de 0,2 A durant 2 secondes.

## Revendications

1. Dispositif de détection et de transmission de l'alarme du rythme cardiaque d'une personne au moyen d'un premier module (12) de détection et d'émission comprenant un appareil portatif à capteur (10, 11) de mesure fixé sur le corps de la personne, et destiné à délivrer un signal de mesure du rythme cardiaque à un convertisseur tension-fréquence (18),
caractérisé en ce que :
- le convertisseur tension-fréquence (18) est connecté à un premier circuit émetteur (20) radioélectrique à très courte portée, destiné à émettre un signal de fréquence cardiaque au moyen d'une première antenne émettrice (22),
- un deuxième module relais (14) est placé à proximité du premier module (12), et comporte un premier circuit récepteur (24) radioélectrique associé à une première antenne réceptrice (26) à très courte portée, susceptible de capter le signal émis par la première antenne émettrice (22), un écran de visualisation (28) pour afficher en lecture directe les pulsations du rythme cardiaque, un dispositif de réglage (30) pour ajuster un seuil haut, et un seuil bas du rythme cardiaque, et un transmetteur (32) capable de délivrer un signal sonore d'alarme dès que le signal de fréquence cardiaque dépasse le seuil haut, ou descend en-dessous du seuil bas,
- un système de télétransmission est logé dans le deuxième module relais 14 et comprend un deuxième circuit émetteur (36) radioélectrique à longue portée, piloté par un détecteur acoustique (34) sensible au signal sonore d'alarme, et associé à une deuxième antenne émettrice (38),
- un troisième module (16) de réception du signal d'alarme, est doté d'un deuxième circuit récepteur (40) radioélectrique à longue portée, associé à une deuxième antenne réceptrice (42), et d'un circuit amplificateur (44) branché à un indicateur (46) sonore ou visuel, ledit troisième module (16) portatif étant sous la surveillance d'une autre personne.

2. Dispositif de détection et de transmission de l'alarme du rythme cardiaque selon la revendication 1, caractérisé en ce que le détecteur acoustique (34) est formé par un microphone polarisé à électret (64) coopérant avec un relais (66) de commande d'un circuit inverseur (70) de polarité associé au deuxième circuit émetteur (36).

3. Dispositif de détection et de transmission de l'alarme du rythme cardiaque selon la revendication 2, caractérisé en ce que le microphone polarisé à électret (64) est inséré à l'entrée d'un circuit de traitement (62) ayant des moyens de réglage du seuil de déclenchement du relais (66).

4. Dispositif de détection et de transmission de l'alarme du rythme cardiaque selon l'une des revendications 1 à 3, caractérisé en ce que le deuxième module relais (14) comporte deux alimentations (31, 39) séparées, l'une (31) étant affectée au premier circuit récepteur (24), et l'autre (39) au système de télétransmission.

5. Dispositif de détection et de transmission de l'alarme du rythme cardiaque selon la revendication 4, caractérisé en ce que la première alimentation (31) comporte un premier régulateur de tension (56), et un accumulateur (58) de secours pour mémoriser les paramètres de réglage des seuils haut et bas, et pour afficher en permanence le rythme cardiaque indépendamment de l'état de la deuxième alimentation (39).

6. Dispositif de détection et de transmission de l'alarme du rythme cardiaque selon l'une des revendications 3 à 5, caractérisé en ce que le relais (66) est connecté dans le circuit collecteur d'un transistor T3 du circuit de traitement (62), et est commuté de la position R de repos vers la position T de travail dès que le signal transmis au microphone (64) dépasse ledit seuil de déclenchement.

7. Dispositif de détection et de transmission de l'alarme du rythme cardiaque selon l'une des revendications 2 à 6, caractérisé en ce que le deuxième module relais (14) est équipé d'un circuit test (70) à bouton de manoeuvre (72) destiné à simuler la commutation du relais (60) pour vérifier l'émission du signal d'alarme à haute fréquence par le deuxième circuit émetteur (36) du système de télétransmission.
